# EUROPEAN PATENT APPLICATION

(11) **EP 2 070 545 A1**
(43) Date of publication of application: **17.06.2009**
(21) Application number: 08021434.9
(22) Date of filing: 10.12.2008
(51) Int. Cl.: A61K 36/534, A61K 36/9066, A61K 36/63, A61P 1/00

(54) **Oral compositions for the prevention and treatment of inflammatory disorders of the colon**

(30) Priority: 11.12.2007 IT MI20072315
(71) Applicant: Bios Line S.p.a., 35020 Ponte S. Nicolo (PD) (IT)
(72) Inventor: Tramonti, Paolo, 35100 Padova (IT)
(74) Representative: Minoja, Fabrizio

(57) **Abstract**

Oral compositions containing mint essential oil, *Curcuma longa* derivatives, *Olea europea* derivatives, and possibly one or more of: N-acetylcysteine, glutathione, ubidecarenone, lactoferrin, carotenoids, polyphenols, vitamin C, vitamin E, extract or derivative of St. John's Wort, kava kava, saffron, valerian, passion-flower, camomile and griffonia for the prevention and treatment of inflammatory disorders of the colon.

## Description

### FIELD OF INVENTION

The present invention relates to oral compositions containing mint essential oil, *Curcuma longa* derivatives, *Olea europea* derivatives, and possibly one or more of: N-acetylcysteine, glutathione, ubidecarenone, lactoferrin, carotenoids, polyphenols, vitamin C, vitamin E, extract or derivative of St. John's Wort, kava kava, saffron, valerian, passionflower, camomile and griffonia.

The compositions according to the invention are useful for the prevention and treatment of inflammatory disorders of the colon. Said formulations can be normal-release, time-dependent, gastroprotected, or preferably colon-specific.

### PRIOR ART

The pharmaceutical development of a product, whether it is a pharmaceutical or merely nutritional product, involves optimising the pharmacological yield of the active ingredients it contains. This is strongly influenced by the timing and methods of release of the active ingredients from the pharmaceutical form used (whether it is a tablet, capsule or other form).

The possibility of modulating their release on the basis of a given pH is particularly significant in this respect. By exploiting the differences in pH between the stomach (approx. 1-1.5), the small intestine (6.8) and the large intestine (7.2), the release can be modulated to ensure that it takes place in the required area. When the release is triggered by a pH of 7.2, it is said to be colon-specific. A colon-specific product is therefore one which only releases its active ingredients in the large intestine (the ascending colon, transverse colon, descending colon, sigmoid colon and rectum). As stated, the specific release of drug in the colon is possible in view of the marked (though numerically small) variation in pH between the small intestine (6.8) and the colon (7.2).

This colon-specific release technology, now also available for dietary preparations due to the use of food-grade ingredients, has numerous potential applications. Some examples are problems like constipation and diarrhoea, disorders such as colitis, and serious diseases like Crohn's disease. Colon-specificity also enables products with a high safety profile to be produced. The colon absorbs no substances apart from water, so this technology can also be effectively used with products which are moderately toxic when absorbed, but have a strong local anti-inflammatory action.

Irritable Bowel Syndrome (IBS) is a disorder that affects the colon. It is commonly called colitis, which refers generically to inflammation of the colon. IBS is the most common type of colitis, and is continually increasing in the Western world.

The causes which may give rise to irritable bowel syndrome are not yet fully understood, although the direct correlation between the disease and long-term conditions of psychological stress, anxiety and agitation is now well established. These psychological conditions appear to be accentuated by a sedentary lifestyle and an irregular diet containing little fibre, both of which are very common in Western society.

The symptoms of IBS are highly varied. The most common are abdominal symptoms such as flatulence and abdominal tension, often associated with depression, anxiety, fatigue, and difficulty in concentrating or performing normal everyday activities. The frequency of bowel movements becomes extremely variable, with an alteration in the consistency of the stools, ranging from obstinate constipation to diarrhoea alternating with constipation, and chronic diarrhoea. On the basis of the symptoms, two types of syndrome can be identified: "spastic colon" and "painless diarrhoea".

In spastic colon syndrome the bowel movements can be highly variable, and patients experience intense pain alternating with remission, associated with constipation or periods of severe diarrhoea. Pain is generally eliminated by evacuation, and is often triggered by swallowing certain foods, which vary from patient to patient. In the "painless diarrhoea" syndrome, urgent diarrhoea occurs, usually during or immediately after meals. In the most serious and persistent forms the patient may also suffer from faecal incontinence and, rarely, nocturnal diarrhoea.

Most of the symptoms of IBS are due to incorrect functioning of the smooth muscles of the abdomen and the enteric nervous system.

The intestinal muscles contract and distend to move food, which travels from the stomach through the intestinal tract to the rectum. These muscles usually move with a coordinated rhythm. In IBS, however, the contractions are longer and stronger than usual. Food is therefore pushed more quickly through the intestine, causing gas formation, swelling and diarrhoea. In other cases, however, the opposite occurs. The passage of food is slower, and the stools become hard and dry. As already stated, the cause(s) of IBS are not fully understood. Some researchers believe that it is due to an alteration in the nerve function that controls the muscle contractions or sensitivity of the intestine. Moreover, as two-thirds of the patients who suffer from this disorder are women, some authors consider that hormonal changes play an important part. In fact, many women find that the symptoms increase during menstruation. Most patients who suffer from IBS experience a considerable increase in their symptoms during stressful events. However, stress can only aggravate the symptoms, but never cause them.

IBS is sometimes triggered by another disorder, such as an acute episode of diarrhoea (gastroenteritis). It can also be triggered by antibiotics, which destroy the normal commensal bacterial flora. Excessive use of laxatives and some anti-diarrhoeal drugs can also contribute to the problem.

No effective cure for IBS has yet been found.

The less serious symptoms of irritable bowel syndrome can usually be kept under effective control by controlling stress and changing diet and lifestyle (physical exercise can be very helpful). In the case of moderate or severe symptoms, however, these precautions are by no means sufficient.

For moderate symptoms, official medicine suggests enriching the diet with fibre supplements such as psyllium or methylcellulose or, alternatively, the use of antidiarrhoeal drugs, such as those based on loperamide, to combat diarrhoea, or laxatives to control constipation. Anticholinergic drugs, which alleviate the painful bowel spasms by acting on the nervous system, are also sometimes used. In the case of symptoms involving pain and depression, tricyclic antidepressants or selective serotonin reuptake inhibitors can be used. Said drugs, as well as alleviating depression, inhibit the activity of the neurones that control the intestine. In the case of diarrhoea with abdominal pain, tricyclic antidepressants such as imipramine and amitriptyline are indicated. Said substances usually involve side effects such as insomnia, nausea, dry mouth and constipation. Conversely, selective serotonin reuptake inhibitors such as fluoxetine (Prozac) and oxetine can have a favourable effect in the case of depression, abdominal pain and constipation, but are not always effective.

The anti-IBS action of 100% pure mint essential oil, characterised by the presence of menthol and carvone, has been known for several years.

To date, no less than 16 clinical trials (double-blind, placebo-controlled) have been published which describe the use of mint essential oil in the treatment of irritable bowel syndrome and dyspepsia. Said studies obviously involved thousands of adult patients. In some of those studies the action of the product on infantile IBS was also evaluated. All those studies demonstrate the great usefulness of the product, which proved effective in reducing irritative and pain symptoms (whether or not diarrhoea was present, and resolving it if present) in 50 to 55% of cases after only a few weeks' treatment. Unfortunately, the percentage does not increase with longer-term use of the product. The use of mint essential oil therefore produces a satisfactory clinical picture in roughly 1 case in 2.

The action mechanism of menthol and the essential oil that contains it is still being investigated. At present, their action in the calcium (Ca++) channels of the intestinal cells seems certain. Their antagonism against the calcium channels may explain the smooth muscle relaxant action demonstrated in clinical practice on patients treated with mint essential oil. The blockade of the calcium pump is believed to inhibit intestinal contractions, causing relaxation and the resumption of more rhythmic, non-spasmodic contractions. Antagonism against the calcium channels does not rule out other possible action mechanisms which may contribute in some way to the anti-IBS efficacy of the product.

However, widespread use of menthol and essential oil has always been limited by their low tolerability. After swallowing, these products cause acid reflux and oesophagitis. The use of microencapsulated and/or gastroprotected mint oil has therefore begun in the last 5 years. However, even at best, gastroprotection does not wholly eliminate the problem of reflux and the consequent oesophagitis. Part of the product flows back through the cardia, causing particular discomfort to the patient.

Especially in paediatric patients (IBS also affects children and adolescents) the problem is strongly felt, and limits nearly all its possible uses.

Turmeric (curcumin) is a food colouring, used to give dishes a yellow colour similar to that of saffron. In the European Union coding system, it is identified as E100. At ambient temperature it appears as a deep yellow-orange crystalline powder. It is poorly soluble in water, but soluble in ethanol and acetic acid.

Curcumin is obtained by extraction with solvent from the rhizome of the *Curcuma longa* (*Curcuma domestica* Valeton) plant, followed by purification by crystallisation. It is accompanied by small amounts of its demethoxy- and bis-demethoxy-derivatives, ie. derivatives which lack one or both -OCH₃ groups. Its identification is based on the behaviour of turmeric in an acid environment: the colour of its solution in ethanol must be yellow with green fluorescence, changing to deep red when concentrated sulphuric acid is added. Curcumin is responsible for the biological effect observed when the native extract of turmeric is used, and possesses choleretic, cholagogic, anti-inflammatory and pancreas-protecting activity. Its oral absorption is modest, but sufficient to cause marked biological activity, which is used nowadays to treat conditions ranging from simple oxidative stress (typical of aging) in the peripheral tissues to cystic fibrosis and Alzheimer's disease. When used by topical application in cosmetic preparations, it has demonstrated a good soothing activity. Its biological action after oral administration is associated with the oral bioavailability of the product which is absorbed intestinally, reaches the liver through the portal circuit, and from there is distributed as a complexed derivative to the peripheral regions, and then eliminated as sulphate or glucuronide through the urine.

An extracted derivative can be obtained from the flesh of the Mediterranean olive (*Olea europea*) which is characterised by the presence of oleuropein and verbascoside, very potent glycosidated antioxidants which, after detachment of the carbohydrate component, give rise to aglycons with an even more marked antioxidant power, such as hydroxytyrosol. After oral administration, it shifts the tissue redox potential and effectively combats oxidation in the cells.

### DESCRIPTION OF THE INVENTION

The present invention relates to oral compositions with colon-specific release containing:
a) mint essential oil;
b) *Curcuma longa* derivatives;
c) *Olea europea* derivatives;
   and possibly one or more of:
d) N-acetylcysteine;
e) glutathione;
f) ubidecarenone;
g) lactoferrin;
h) carotenoids;
i) polyphenols;
j) vitamin C;
k) vitamin E;
l) St. John's Wort extract or a derivative thereof;
m) kava kava extract or a derivative thereof;
n) valerian extract or a derivative thereof;
o) saffron extract or a derivative thereof;
p) camomile extract or a derivative thereof;
q) passionflower extract or a derivative thereof;
r) griffonia extract or a derivative thereof;
for the prevention and treatment of inflammatory disorders of the colon.

The oral compositions according to the invention will preferably be the colon-specific release type for two reasons: firstly, to convey the substances to the colon, an area where absorption does not take place and the active ingredients can therefore perform their local antioxidant/anti-inflammatory action (components a-k) and control the intestinal neurotransmitters (components 1-r); and secondly, to prevent the risk of adverse gastrointestinal effects.

When the compositions according to the invention are colon-specific, they present numerous advantages:
i) The problem of reflux and oesophagitis induced by mint oil (or its active ingredients, such as menthol, menthone and methyl acetate), which still arises in the gastroprotected formulations, is now solved, because said active ingredients do not come into contact with the gastric environment or the small intestine.
ii) The problem of slight oral absorption of curcumin (and the extracted derivative of *Curcuma longa,* from which it is obtained) is eliminated, because the active ingredient is not absorbed, but is unchanged when it reaches the colon, where it is not absorbed but performs its anti-inflammatory action locally.
iii) It has also surprisingly been found that the addition of extracted derivatives of *Curcuma longa* and *Olea europea,* which have never been used before in inflammatory disorders of the colon, unexpectedly enhances the action of mint essential oil, the action observed being the result of an evident synergy between the various active ingredients rather than a cumulative effect thereof. The mixture of components markedly reduces the time normally expected to obtain an evident response (from 6-7 to 4-5 days), with an efficacy rate ranging between 85% and 95%.

The compositions according to the invention are characterised by a considerable anti-inflammatory action which provides evident benefits in all inflammatory conditions of the colon with various etiologies (ranging from simple colitis with a psychosomatic basis to well-known disorders like irritable bowel syndrome).

According to the invention, the extracted derivatives of *Curcuma longa* and *Olea europea* will be present in the compositions in the form of a dried extract, preferably standardised, or in the form of pure active ingredients which are present in the extract and identified (e.g. curcumin, oleuropein, verbascoside and hydroxytyrosol), in free form, in the form of complexes with phospholipids, or complexed in the form of co-ground products or ter-clathrates.

According to a preferred aspect, the compositions according to the invention will contain the derivatives of *Curcuma longa* and *Olea europea* in the form of active ingredients complexed with phospholipids or in the form of co-ground products or ter-clathrates.

According to a preferred aspect, the compositions according to the invention will contain an extracted derivative of *Olea europea* which has a total polyphenol content of not less than 20%, and contains 3-5% verbascoside and 0.5-1.5% hydroxytyrosol. Pharmacological experiments with said compositions have demonstrated a particularly effective anti-inflammatory effect on the colon.

According to the invention, the carotenoids present as optional components will preferably be chosen from lycopene, lutein, zeaxanthin, astaxanthin and beta-carotene.

According to the invention, the polyphenols present as optional components will preferably be chosen from polyphenols obtained from vines, tea, bilberry and milk thistle.

Optional components d)-k), characterised by a very high antioxidant action profile which is normally performed in the peripheral tissue after intestinal absorption, are unchanged when they reach the colon, where they effectively help to combat the symptoms of IBS and, more generally, all the irritative and inflammatory problems of the colon area, including proctitis.

Optional components 1)-r), characterised by a very high relaxing, sedative, tranquillising and anti-panic power, operate as sedatives in the normal-release and gastroprotected formulations, and therefore reduce the psychosomatic component of IBS; in the colon-specific formulations they operate as local modulators of the intestinal neurotransmitters.

The compositions according to the invention will contain the various components in the following dose intervals:
a) mint essential oil: 10-1000 mg/day;
b) standardised dried extract of *Curcuma longa*: 10-2000 mg/day, or curcumin: 0.01-100 mg/day;
c) standardised dried extract of *Olea europea*: 10-2000 mg/day; or oleuropein and/or verbascoside and/or hydroxytyrosol: 0.1-500 mg/day;
   and possibly one or more of:
d) N-acetylcysteine: 0.1-1200 mg/day;
e) glutathione: 0.1-250 mg/day;
f) ubidecarenone: 0.1-500 mg;
g) lactoferrin: 0.1-250 mg/day;
h) carotenoids: 0.1-25 mg/day;
i) polyphenols: 0.1-3000 mg/day;
j) vitamin C: 0.1-500 mg/day;
k) vitamin E: 0.1-500 mg/day;
l) St. John's Wort extract or a derivative thereof; 0.1-7200 mg/day;
m) kava kava extract or a derivative thereof; 0.1-500 mg/day;
n) valerian extract or a derivative thereof; 0.1-1500 mg/day;
o) saffron extract or a derivative thereof; 0.1-2500 mg/day;
p) camomile extract or a derivative thereof; 0.1-2500 mg/day;
q) passionflower extract or a derivative thereof; 0.1-2500 mg/day;
r) griffonia extract or a derivative thereof; 0.1-2500 mg/day.

The compositions according to the invention may be presented as solid oral forms such as soft or hard gelatin capsules, granulates, sachets, tablets and the like, with normal release, gastroprotected or colon-specific release effected by coating with suitable film-forming solutions.

According to the invention, the solid oral forms will have pH-dependent (colon-specific) release due to the use of two particular film-forming layers of coating, used at the concentration of 11% and 15%.

The first film-forming solution, which will constitute the inner coating, may consist of an aqueous solution containing a structural agent, an alkalising agent and a plasticiser. Typically, 87.2% shellac will be used as structural agent, 3.3% ammonium carbonate as alkalising agent, and 9.5% triethyl citrate as plasticiser. Said first coating will protect the product from dissolving at a pH of 6.8 (the intestinal pH typical of the small intestine).

The second film-forming solution, which will constitute the outer coating, can include the same structural, alkalising and plasticising agents, in different percentages, typically 84.2%, 6.3% and 9.5% respectively. Said second coating protects the product from dissolving at pH of 1.0-1.5 (typically gastric pH).

This double coating only allows the compositions according to the invention to dissolve at pH 7.2 (typical of the colon), where they can release the various active ingredients, whose action will be solely colon-specific and local.

A double film-forming solution is thus obtained which, when suitably layered on the core of the tablet or capsule, leads to colon-specific release of the active ingredients.

The use of the second film-forming solution alone will obviously only provide gastroprotection of the finished product.

An example of preparation of soft gelatin capsules with colon-specific release, according to a preferred aspect of the invention, is given below.

Soft gelatin capsules with colon-specific release are prepared as follows:
A first aqueous solution containing 87.2% shellac, 3.3% ammonium carbonate and 9.5% triethyl citrate is sprayed onto the soft gelatin capsule at ambient temperature, but in a heated coating pan; when drying is finished, a second film-forming solution at the concentrations of 84.2%, 6.3% and 9.5% respectively is sprayed on.
Alternatively, soft gelatin capsules can be prepared with Eudragit (S). Chemically, said substance corresponds to methacrylic acid copolymer type B according to USP/NF. The use of said compound only allows the product to permeate into the stomach juices at a pH greater than 7.

### FORMULATION EXAMPLES

### EXAMPLE 1

| Ingredient | Amount in mg/day |
|---|---|
| Mint essential oil | 400.00 |
| *Curcuma longa* extract | 100.00 |
| *Olea europea* extract | 50.00 |
| Linseed oil | 111.85 |
| Glycerol monostearate | 30.20 |
| Soya lecithin | 7.95 |
| Gelatin | 138.27 |
| Glycerol | 62.07 |
| E171 colouring | 4.89 |
| E172 yellow colouring | 0.53 |
| E172 red colouring | 1.22 |
| Sepifilm SN | 24.24 |
| Shellac | 36.30 |
| Ammonium carbonate | 2.72 |
| Triethyl citrate | 4.08 |
| Purified water | 388.21 |

### EXAMPLE 2

| Ingredient | Amount in mg/day |
|---|---|
| Mint essential oil | 250.00 |
| *Olea europea* extract | 100.00 |
| *Curcuma longa* extract | 100.00 |
| Glutathione | 30.00 |
| CoQ10 | 10.00 |
| Linseed oil | 111.85 |
| Glycerol monostearate | 30.20 |
| Soya lecithin | 7.95 |
| Gelatin | 138.27 |
| Glycerol | 62.07 |
| E171 colouring | 4.89 |
| E172 yellow colouring | 0.53 |
| E 172 red colouring | 1.22 |
| Sepifilm SN | 24.24 |
| Shellac | 36.30 |
| Ammonium carbonate | 2.72 |
| Triethyl citrate | 4.08 |
| Purified water | 388.21 |

### EXAMPLE 3

| Ingredient | Amount in mg/day |
|---|---|
| Mint essential oil | 200.00 |
| Curcumin | 50.00 |
| *Olea europea* extract | 150.00 |
| Linseed oil | 111.85 |
| Glycerol monostearate | 30.20 |
| Soya lecithin | 7.95 |
| Gelatin | 138.27 |
| Glycerol | 62.07 |
| E171 colouring | 4.89 |
| E172 yellow colouring | 0.53 |
| E172 red colouring | 1.22 |
| Sepifilm SN | 24.24 |
| Shellac | 36.30 |
| Ammonium carbonate | 2.72 |
| Triethyl citrate | 4.08 |
| Purified water | 388.21 |

### EXAMPLE 4

| Ingredient | Amount in mg/day |
|---|---|
| Mint essential oil | 250.00 |
| Leucocyanidin from *Vitis vinifera* | 100.00 |
| *Olea europea* extract | 50.00 |
| *Curcuma longa* extract | 50.00 |
| Lycopene from *L. aesculentum* | 10.00 |
| Linseed oil | 111.85 |
| Glycerol monostearate | 30.20 |
| Soya lecithin | 7.95 |
| Gelatin | 138.27 |
| Glycerol | 62.07 |
| E171 colouring | 4.89 |
| E172 yellow colouring | 0.53 |
| E 172 red colouring | 1.22 |
| Sepifilm SN | 24.24 |
| Shellac | 36.30 |
| Ammonium carbonate | 2.72 |
| Triethyl citrate | 4.08 |
| Purified water | 388.21 |

### EXAMPLE 5

| Ingredient | Amount in mg/day |
|---|---|
| Mint essential oil | 400.00 |
| *Olea europea* extract | 100.00 |
| *Curcuma longa* extract | 100.00 |
| Saffron extract | 50.00 |
| Camomile extract | 100.00 |
| Linseed oil | 111.85 |
| Glycerol monostearate | 30.20 |
| Soya lecithin | 7.95 |
| Gelatin | 138.27 |
| Glycerol | 62.07 |
| E171 colouring | 4.89 |
| E172 yellow colouring | 0.53 |
| E 172 red colouring | 1.22 |
| Sepifilm SN | 24.24 |
| Shellac | 36.30 |
| Ammonium carbonate | 2.72 |
| Triethyl citrate | 4.08 |
| Purified water | 388.21 |

### EXAMPLE 6

| Ingredient | Amount in mg/day |
|---|---|
| Mint essential oil | 250.00 |
| *Olea europea* extract | 100.00 |
| *Curcuma longa* extract | 100.00 |
| St. John's Wort extract | 300.00 |
| Saffron extract | 150.00 |
| Linseed oil | 111.85 |
| Glycerol monostearate | 30.20 |
| Soya lecithin | 7.95 |
| Gelatin | 138.27 |
| Glycerol | 62.07 |
| E171 colouring | 4.89 |
| E172 yellow colouring | 0.53 |
| E 172 red colouring | 1.22 |
| Sepifilm SN | 24.24 |
| Shellac | 36.30 |
| Ammonium carbonate | 2.72 |
| Triethyl citrate | 4.08 |
| Purified water | 388.21 |

### EXAMPLE 7

| Ingredient | Amount in mg/day |
|---|---|
| Mint essential oil | 250.00 |
| *Olea europea* extract | 100.00 |
| *Curcuma longa* extract | 100.00 |
| Glutathione | 30.00 |
| Saffron extract | 250.00 |
| Linseed oil | 111.85 |
| Glycerol monostearate | 30.20 |
| Soya lecithin | 7.95 |
| Gelatin | 138.27 |
| Glycerol | 62.07 |
| E171 colouring | 4.89 |
| E172 yellow colouring | 0.53 |
| E172 red colouring | 1.22 |
| Sepifilm SN | 24.24 |
| Shellac | 50.50 |
| Purified water | 388.21 |

### EXAMPLE 8

| Ingredient | Amount in mg/day |
|---|---|
| Mint essential oil | 150.00 |
| *Olea europea* extract | 100.00 |
| *Curcuma longa* extract | 100.00 |
| Glutathione | 30.00 |
| Saffron extract | 250.00 |
| Linseed oil | 111.85 |
| Glycerol monostearate | 30.20 |
| Soya lecithin | 7.95 |
| Gelatin | 138.27 |
| Glycerol | 62.07 |
| E171 colouring | 4.89 |
| E172 yellow colouring | 0.53 |
| E 172 red colouring | 1.22 |

## Claims

1. Oral compositions containing:
a) mint essential oil;
b) *Curcuma longa* derivatives;
c) *Olea europea* derivatives;
and possibly one or more of:
d) N-acetylcysteine;
e) glutathione;
f) ubidecarenone;
g) lactoferrin;
h) carotenoids;
i) polyphenols;
j) vitamin C;
k) vitamin E;
l) St. John's Wort extract or a derivative thereof;
m) kava kava extract or a derivative thereof;
n) valerian extract or a derivative thereof;
o) saffron extract or a derivative thereof;
p) camomile extract or a derivative thereof;
q) passionflower extract or a derivative thereof;
r) griffonia extract or a derivative thereof;

2. Compositions as claimed in claim 1, wherein derivatives extracted from *Curcuma longa* are present in the form of a dried extract or as curcumin.

3. Compositions as claimed in claim 2, wherein the dried extract of *Curcuma longa* is standardised.

4. Compositions as claimed in claim 2, wherein the curcumin is present in free form or in the form of complexes with phospholipids, co-ground products or ter-clathrates.

5. Compositions as claimed in claim 1, wherein the extracted derivatives of *Olea europea* are present in the form of a dried extract, or as oleuropein, verbascoside and hydroxytyrosol.

6. Compositions as claimed in claim 5, wherein the dried extract of *Olea europea* is standardised, **characterised by** a total polyphenol content of not less than 20%, and contains 3-5% verbascoside and 0.5-1.5% hydroxytyrosol.

7. Compositions as claimed in claim 5, wherein oleuropein, verbascoside and hydroxytyrosol are present in free form or in the form of complexes with phospholipids, co-ground products or ter-clathrates.

8. Compositions as claimed in claim 1 and 2, wherein the carotenoids are chosen from lycopene, lutein, zeaxanthin, astaxanthin and beta-carotene.

9. Compositions as claimed in claims 1 and 2, wherein the polyphenols are chosen from polyphenols obtained from vines, tea, bilberry and milk thistle.

10. Compositions as claimed in the preceding claims, containing the various ingredients in the following dose intervals:
a) mint essential oil: 10-1000 mg/day;
b) standardised dried extract of *Curcuma longa*: 10-2000 mg/day, or curcumin: 0.01-100 mg/day;
c) dried standardised extract of *Olea europea*: 10-2000 mg/day; or oleuropein and/or verbascoside and/or hydroxytyrosol: 0.1-500 mg/day;
and possibly one or more of:
d) N-acetylcysteine: 0.1-1200 mg/day;
e) glutathione: 0.1-250 mg/day;
f) ubidecarenone: 0.1-500 mg;
g) lactoferrin: 0.1-250 mg/day;
h) carotenoids: 0.1-25 mg/day;
i) polyphenols: 0.1-3000 mg/day
j) vitamin C: 0.1-500 mg/day;
k) vitamin E: 0.1-500 mg/day;
l) St. John's Wort extract or a derivative thereof; 0.1-7200 mg/day;
m) kava kava extract or a derivative thereof; 0.1-500 mg/day;
n) valerian extract or a derivative thereof; 0.1-1500 mg/day;
o) saffron extract or a derivative thereof; 0.1-2500 mg/day;
p) camomile extract or a derivative thereof; 0.1-2500 mg/day;
q) passionflower extract or a derivative thereof; 0.1-2500 mg/day;
r) griffonia extract or a derivative thereof; 0.1-2500 mg/day.

11. Compositions as claimed in any of the preceding claims, with normal, time-dependent, gastroprotected or colon-specific release.

12. Compositions as claimed in claim 11 with colon-specific release.

13. Compositions as claimed in the preceding claims, as solid oral forms chosen from soft or hard gelatin capsules, granulates, sachets or tablets.

14. The use of:
a) mint essential oil;
b) *Curcuma longa* derivatives;
c) *Olea europea* derivatives;
and possibly one or more of:
d) N-acetylcysteine;
e) glutathione;
f) ubidecarenone;
g) lactoferrin;
h) carotenoids;
i) polyphenols;
j) vitamin C;
k) vitamin E;
l) St. John's Wort extract or a derivative thereof;
m) kava kava extract or a derivative thereof;
n) valerian extract or a derivative thereof;
o) saffron extract or a derivative thereof;
p) camomile extract or a derivative thereof;
q) passionflower extract or a derivative thereof;
r) griffonia extract or a derivative thereof;
for the preparation oral compositions for the prevention and treatment of inflammatory disorders of the colon.
